# EUROPÄISCHE PATENTSCHRIFT

(11) **EP 2 012 856 B1**
(45) Veröffentlichungstag und Bekanntmachung des Hinweises auf die Patenterteilung: **08.09.2010**
(21) Anmeldenummer: 07724366.5
(22) Anmeldetag: 19.04.2007
(51) Int. Cl.: A61M 5/50

(54) **SPRITZENZYLINDER**
SYRINGE CYLINDER
CYLINDRE DE SERINGUE

(30) Priorität: 21.04.2006 DE 102006018651
(43) Veröffentlichungstag der Anmeldung: 14.01.2009
(73) Patentinhaber: Arzneimittel GmbH Apotheker Vetter & Co. Ravensburg, 88212 Ravensburg (DE)
(72) Erfinder: BÖBST, Benjamin, 88441 Mittelbiberach (DE); PETERS, Dirk, 88212 Ravensburg (DE); BOETTGER, Frank, 88214 Ravensburg (DE)
(74) Vertreter: Gleiss, Alf-Olav
(86) Internationale Anmeldenummer: PCT/EP2007/003429
(87) Internationale Veröffentlichungsnummer: WO 2007/121915

(56) Entgegenhaltungen:
- US-A- 4 392 852
- US-A- 5 338 311

## Beschreibung

Die Erfindung betrifft einen Spritzenzylinder mit einem Innenraum gemäß Oberbegriff des Anspruchs 1.

Spritzenzylinder der hier angesprochenen Art sind bekannt. Sie weisen einen Innenraum mit einem Stopfen auf, der einen Wirkstoffraum nach außen abgrenzt. In den Wirkstoffraum kann sich eine Luftblase befinden, die bei Temperaturschwankungen und insbesondere bei Druckunterschieden während eines Lufttransports des Spritzenzylinder sich ausdehnen und zusammenziehen kann. Durch Druckunterschiede kann sich der Stopfen im Innenraum des Spritzenzylinders verlagern. Dabei kann er Bereiche des Innenraums erreichen, die nicht oder nicht ausreichend steril sind. Bei einer Rückverlagerung des Stopfens bei einer Veränderung der Temperatur oder bei einem Druckabfall kann dadurch der Wirkstoffraum verunreinigt werden. Um vor Einsatz eines Spritzenzylinders und vor Verwendung eines im Wirkstoffraum untergebrachten Medikaments feststellen zu können, ob der Stopfen über einen maximal zulässigen Weg verschoben wurde, ist es erforderlich, den von dem Stopfen zurückgelegten Weg zu erfassen. Es ist bekannt, den Innenraum eines Spritzenzylinders auf der den Wirkstoffraum abgewandten Seite eines Stopfens mit Graphitpulver zu versehen. Das an der Innenwand des Spritzenzylinders anhaftende Graphitpulver wird bei einer Verlagerung des Stopfens verschoben, sodass bei einer Rückverlagerung des Stopfens ein pulverfreier Bereich der Innenwand des Spritzenzylinders sichtbar wird. Um eine Verlagerung des Stopfens beispielsweise während eines Lufttransports feststellen zu können, wurde bei herkömmlichen Spritzenzylindern der graphitpulverfreie Bereich erfasst. Ab einer gewissen, in Längsrichtung des Spritzenzylinders gemessenen Breite dieses Bereichs konnte man davon ausgehen, dass der Stopfen nicht oder nicht ausreichend sterilisierte Bereiche des Innenraums erreicht hatte und der Wirkstoffraum verunreinigt sein konnte. Derartige Spritzenzylinder konnten dann ausgesondert werden.

Es hat sich gezeigt, dass die Reibungskräfte zwischen Stopfen und Innenwand des Spritzenzylinders durch das Graphitpulver verändert wurden. Dies konnte zu Verfälschungen des Messergebnisses führen. Überdies konnte Graphitpulver in den Bereich zurückgelangen, der von dem Stopfen während einer Verlagerung überstrichen wurde. Damit war nicht mit eindeutiger Sicherheit erkennbar, ob ein Stopfen tatsächlich nicht sterile Bereiche erreicht hatte oder nicht.

Aus der US 4 392 852 A geht ein gattungsgemäßer Spritzenzylinder hervor, der einen Innenraum aufweist, in dem ein verlagerbarer Stopfen vorgesehen ist, der einen Wirkstoffraum abgrenzt. Der Spritzenzylinder weist eine Anzeigeeinrichtung auf, welche eine in einer Richtung erfolgende Längsbewegung des Stopfens anzeigt. Die Anzeigeeinrichtung ist durch ein an der Innenwand des Spritzenzylinders angebrachtes, beim Überfahren durch den Stopfen abreibbares Material gebildet, so dass auch bei einer Rückverlagerung des Stopfens in seine Ausgangsposition dessen Verlagerungsweg erkennbar ist.

Aufgabe der Erfindung ist es, einen Spritzenzylinder zur Verfügung zu stellen, bei dem der Verlagerungsweg des Stopfens eineindeutig identifizierbar ist.

Zur Lösung dieser Aufgabe wird ein Spritzenzylinder vorgeschlagen, der die in Anspruch 1 genannten Merkmale aufweist. Der Spritzenzylinder weist einen Innenraum mit einem Stopfen auf, der einen Bereich des Innenraums abtrennt, in den ein Wirkstoff eingebracht ist. Es wird damit ein Wirkstoffraum geschaffen, der gegenüber der Umgebung durch den Stopfen abgeschlossen ist. Der Spitzenzylinder weist eine Anzeigeeinrichtung auf, welche dazu dient, eine in einer Richtung erfolgende Längsbewegung des Stopfens innerhalb des Innenraums eineindeutig anzuzeigen. Sollte also durch Temperatur- oder insbesondere Druckunterschiede der Stopfen sich von dem Wirkstoffraum wegbewegt haben, so zeigt die Anzeigeeinrichtung den Verlagerungsweg eineindeutig an. Auch wenn der Stopfen sich also zurückverlagert, ist zur Qualitätskontrolle des Spritzenzylinders der Verlagerungsweg stets eindeutig erkennbar. Der Spritzenzylinder zeichnet sich dadurch aus, dass die Anzeigeeinrichtung ein Anzeigeelement aufweist, das im Innenraum des Spritzenzylinders gegen eine Reibungskraft beweglich ist, wobei die Reibungskraft so ausgelegt ist, dass sich der Stopfen bei einem Überdruck im Wirkstoffraum mit dem Anzeigeelement in Längsrichtung des Spritzenzylinders verlagern kann, und dass das Anzeigeelement bei Rückverlagerung des Stopfens lagefest im Innenraum verbleibt.

Das im Innenraum des Spritzenzylinders beweglich angeordnete Anzeigeelement ist also so ausgebildet, dass sein Außendurchmesser an den Innendurchmesser des Innenraums angepasst ist. Auch die Materialpaarung des Materials des Spritzenzylinders und das des Anzeigeelements wird aufeinander abgestimmt, um Reibungskräfte zwischen Anzeigeelement und Innenwand des Spritzenzylinders aufzubauen. Diese werden so ausgelegt, dass das Anzeigeelement bei Verlagerung des Stopfens im Innenraum des Spritzenzylinders von diesem in einer Richtung verlagerbar ist, nämlich in einer Richtung, die vom Wirkstoffraum wegführt. Wird der Wirkstoffraum mehrfach Temperatur- oder Druckunterschieden unterworfen, so kann sich der Stopfen entsprechend oft von dem Wirkstoffraum wegbewegen und dabei das Anzeigeelement verlagern. Der Stopfen ist mit dem Anzeigeelement nicht gekoppelt, so dass bei einer Rückverlagerung desselben das Anzeigeelement von den Reibungskräften in einer einmal eingenommenen Position gehalten wird. Bei einer Rückverlagerung des Stopfens verbleibt also das Anzeigeelement lagefest im Innenraum des Spritzenzylinders. Damit ist die maximale Verlagerung des Stopfens anhand der Position des Anzeigeelements eineindeutig ermittelbar.

Weitere Ausgestaltungen ergeben sich aus den Unteransprüchen.

Die Erfindung wird im Folgenden anhand der Zeichnungen näher erläutert. Es zeigen:
- Figuren 1 bis 3: eine Prinzipskizze eines Spritzenzylinders in Teil- längsschnitt und
- Figur 4: ein Anzeigeelement in perspektivischer Darstel- lung.

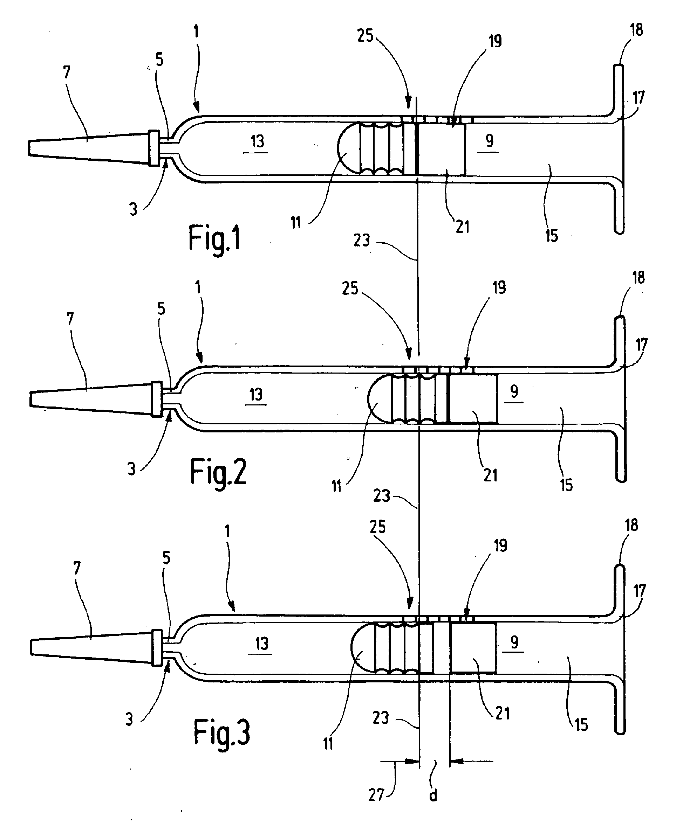

Figur 1 zeigt einen Spritzenzylinder 1 im Längsschnitt. Er weist ein erstes Ende 3 mit einem Nadelansatz 5 auf, auf den eine Kappe 7 aufgesetzt ist.

Der Innenraum 9 des Spritzenzylinders 1 wird durch einen in Längsrichtung des Spritzenzylinders 1 verlagerbaren Stopfen 11 in zwei Bereiche geteilt: Links von dem Stopfen 11 ist ein abgeschlossener Bereich vorhanden, in den ein Wirkstoff einbringbar ist, und der im Folgenden daher als Wirkstoffraum 13 bezeichnet wird. Rechts von dem Stopfen 11 ist ein gegenüber der Umgebung offener Raum 15 vorhanden, der daher nicht steril ist.

An den dem ersten Ende 3 gegenüberliegenden zweiten Ende 17 des Spritzenzylinders 1 ist ein Vorsprung vorgesehen, der als Fingerauflage 18 dienen kann oder zur Befestigung einer Fingerauflage 18.

Der Spritzenzylinder 1 weist eine Anzeigeeinrichtung 19 auf, die hier ein Anzeigeelement 21 umfasst. Dieses ist auf der dem Wirkstoffraum 13 abgewandten Seite des Stopfens 11 im Raum 15 untergebracht und in Längsrichtung des Spritzenzylinders 1 in diesem Raum verlagerbar.

Der Außendurchmesser des Anzeigeelements 21 ist so auf den Innendurchmesser des Raums 15 abgestimmt, dass es nur unter Überwindung einer gewissen Reibungskraft im Innenraum 9 verlagerbar ist. In Figur 1 befindet sich der Stopfen 11 in einer Ausgangsposition, die dieser beispielsweise nach dem Befüllen des Wirkstoffraums 13 unter Atmosphärendruck einnimmt. Diese Position ist durch eine senkrecht zur Längserstreckung des Spritzenzylinders 1 eingezeichnete Hilfslinie 23 angedeutet, an der das rechte Ende des Stopfens 11 anliegt, außerdem das linke Ende des Anzeigeelements 21. Der Stopfen 11 und das Anzeigeelement 21 liegen also in der Ausgangssituation vorzugsweise aneinander an. In Figur 1 ist noch angedeutet, dass der Spritzenzylinder 1 eine Messeinrichtung, vorzugsweise eine Skala 25, aufweist.

Figur 2 zeigt den in Figur 1 wiedergegebenen Spritzenzylinder 1. Gleiche Teile sind mit gleichen Bezugsziffern versehen, sodass insofern auf die Beschreibung zu Figur 1 verwiesen wird.

Der einzige Unterschied zur Darstellung gemäß Figur 1 beruht darin, dass der Stopfen 11 gegenüber Figur 1 nach rechts verlagert wurde. Das rechte Ende des Stopfens 11 liegt also hier rechts von der Hilfslinie 23.

Da das Anzeigeelement 21 am Stopfen 11 in der Ausgangslage gemäß Figur 1 anlag, wird es bei einer Verlagerung des Stopfens 11 nach rechts über die Hilfslinie 23 hinaus ebenfalls nach rechts durch Überwindung der Reibungskräfte zwischen dem Anzeigeelement 21 und der Innenwand des Spritzenzylinders 1 bewegt.

Eine Verlagerung des Stopfens 11 nach rechts kann dadurch eintreten, dass die Temperatur im Wirkstoffraum 13 erhöht wird, sodass dort ein Überdruck entsteht; andererseits insbesondere dadurch, dass der Spritzenzylinder 1 in einen Bereich gebracht wird, dessen Druck unterhalb des Atmosphärendrucks liegt. Dies geschieht beispielsweise bei einem Lufttransport des Spritzenzylinders 1. Durch den dadurch gegebenen Überdruck im Wirkstoffraum 13 wird der Stopfen 11 dann gegenüber der Hilfslinie 23 nach rechts verlagert, wie dies aus Figur 2 ersichtlich ist.

Figur 3 zeigt den Spritzenzylinder 1 mit einem rückverlagerten Stopfen 11. Teile, die mit denen gemäß Figur 1 und 2 übereinstimmen, sind mit gleichen Bezugsziffern versehen, es wird also auf die vorangegangene Beschreibung verwiesen, um Wiederholungen zu vermeiden.

In Figur 3 ist der Stopfen 11 gegenüber der in Figur 2 dargestellten Position nach links verlagert. Da er so ausgelegt ist, dass er nur bei Überwindung von Reibungskräften im Inneren des Spritzenzylinders 1 verlagerbar ist, wird er auch dann, wenn der Spritzenzylinder 1 unter Atmosphärendruck ist, nicht mehr in die in Figur 1 dargestellte Ausgangsposition zurückgelangen. Dies wird insofern deutlich, als er gegenüber der Hilfslinie 23 immer noch etwas nach rechts verlagert ist, allerdings nicht so weit, wie dies in Figur 2 dargestellt ist.

Da das Anzeigeelement 21 nur unter Überwindung von Reibungskräften im Innenraum 9 des Spritzenzylinders 1 verlagerbar und nicht mit dem Stopfen 11 gekoppelt ist, verbleibt dieses in der in Figur 2 dargestellten Position auch dann, wenn, wie in Figur 3 dargestellt, der Stopfen 11 nach links in Richtung auf das erste Ende 3 des Spritzenzylinders 1 zurückverlagert wird.

Im Folgenden wird auf die Funktion der Anzeigeeinrichtung 19 des Spritzenzylinders 1 näher eingegangen:

In Zusammenhang mit den Figuren 1 bis 3 wurde deutlich, dass der Stopfen 11 aus einer Ausgangsposition gemäß Figur 1, die durch die Hilfslinie 23 gekennzeichnet ist, verlagert werden kann. Bei einem Überdruck im Wirkstoffraum 13 gegenüber einem Druck im Raum 15 wird der Stopfen 11 gegenüber der Hilfslinie 23 nach rechts verlagert, sobald die Reibungskräfte zwischen dem Stopfen 11 und der Innenwand des Spritzenzylinders 1 überwunden werden. Bei einer Verlagerung nach rechts, wie sie in Figur 2 dargestellt ist, wird auch das Anzeigeelement 21 der Anzeigeeinrichtung 19 nach rechts verlagert, das bereits in der Ausgangsposition nach Figur 1 an der rechten Seite des Stopfens 11, also an der dem Wirkstoffraum 13 abgewandten Seite, anliegt. Die Reibungskräfte zwischen dem Anzeigeelement 21 und der Innenwand des Spritzenzylinders 1 sind so gering, dass eine Verlagerung des Anzeigeelements 21 durch die Kraft des Stopfens 11 nach rechts über die Hilfslinie 23 hinaus möglich ist. Andererseits sind die Reibungskräfte so hoch, dass das Anzeigeelement 21, das mit dem Stopfen 11 nicht gekoppelt ist, sich aus der in Figur 2 gegebenen Position nicht nach links zurückbewegt, wenn sich der Stopfen 11 gemäß Figur 3 gegenüber der Hilfslinie 23 nach links zurückverlagert.

Durch einen Doppelpfeil ist angedeutet, dass die linke Kante des Anzeigeelements 21 auch bei einer Rückverlagerung des Stopfens 11 in einem Abstand d zur Hilfslinie 23 angeordnet bleibt.

Der Abstand d kann anhand der Skala 25 bestimmt werden.

Grundsätzlich wird bei Spritzenzylindern der hier angesprochenen Art eine erste dem Wirkstoffraum 13 zugeordnete Zone definiert, die nach dem Befüllen des Spritzenzylinders 1 steril ist. Wird der Stopfen 11 über diese Zone hinaus nach rechts verlagert, also gegenüber der hier eingezeichneten Hilfslinie 23, gelangt er schließlich in Bereiche, in denen der Innenraum 9 des Spritzenzylinders 1 nicht mehr steril ist. Bei einer Rückverlagerung des Stopfens 11 kann der Wirkstoff im Wirkstoffraum 13 dann verunreinigt werden.

Wird also der Stopfen 11 über einen definierten Abstand d hinaus nach rechts verlagert, was durch das Anzeigeelement 21 eineindeutig angezeigt wird, kann ein Verwender den Spritzenzylinder 1 verwerfen, um eine Gefährdung eines Patienten auszuschließen.

Da das Anzeigeelement 21 aufgrund der Reibungskräfte gegenüber der Innenwand des Spritzenzylinders 1 aus seiner in Figur 2 eingenommenen Position nicht zurückverlagert wird, was aus Figur 3 ersichtlich ist, kann die maximale Verlagerung des Stopfens 11 nach rechts über die Hilfslinie 23 hinaus eineindeutig angezeigt werden.

Aus den Erläuterungen zu den Figuren 1 bis 3 wird deutlich, dass für die Grundfunktion der Anzeigeeinrichtung 19 die konkrete Ausgestaltung des Anzeigeelements 21 von untergeordneter Bedeutung ist: Entscheidend ist allein, dass das Anzeigeelement 21 bei einer Verlagerung des Stopfens 11 in einer Richtung, nämlich nach rechts über die Hilfslinie 23 hinaus, im Innenraum 9 des Spritzenzylinders 1 verlagert werden kann. Während einer entgegengesetzten Bewegung des Stopfens 11 soll sich das Anzeigeelement 21 aber aufgrund der Reibungskräfte in einer einmal gegebenen Position halten, sodass die maximale Verlagerung des Stopfens 11 stets eindeutig erkennbar bleibt.

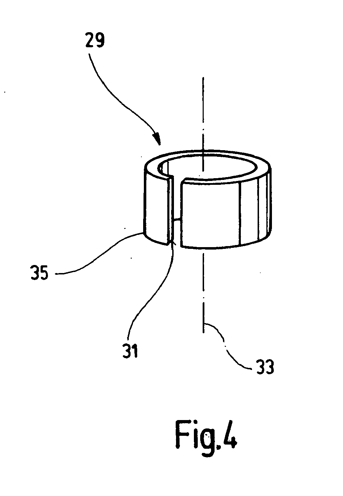

Ein Ausführungsbeispiel des Anzeigeelements 21 ist in Figur 4 dargestellt. Diese zeigt ein als Ring 29 ausgebildetes Anzeigeelement, das vorzugsweise einen in Längsrichtung des Spritzenzylinders 1 verlaufenden Schlitz 31 aufweist. Dieser erhöht die Elastizität des Rings 29. Sein Außendurchmesser ist in entspanntem Zustand etwas größer als der Innendurchmesser des Innenraums 9 des Spritzenzylinders 1. Wird also der Ring 29 in den Innenraum 9 eingeführt, wird er etwas komprimiert, sodass Reibungskräfte zwischen der Innenwand des Innenraums 9 und der Außenfläche des Rings 29 aufgebaut werden.

Die Reibungskräfte können beispielsweise durch die Wahl des Materials des Rings 29 eingestellt werden. Dieser kann beispielsweise Teflon auf seiner Umfangsfläche aufweisen oder aus diesem Material bestehen. Das Material des Anzeigeelements 21 wird auf das Material des Spritzenzylinders 1 abgestimmt, der aus Glas, Kunststoff oder dergleichen bestehen kann.

Zur Einstellung der Reibungskräfte können auch auf der Umfangsfläche des Rings 29 in Umfangsfläche oder in Längsrichtung des Spritzenzylinders 1 verlaufende Rippen vorgesehen werden. Dabei können diese aus einem anderen Material bestehen als der Grundkörper des Rings 29.

Die Reibungskräfte können auch dadurch eingestellt werden, dass die Dicke des Rings 29 und die Breite des Schlitzes 31 variiert werden.

Schließlich ist es noch möglich, auf der Außenfläche des Rings 29 eine oder mehrere unter einem Winkel zur Mittelachse 33 des Rings 29 geneigte Lippe 35 vorzusehen, die mit der Mittelachse 33 einen Winkel α einschließt, der sich - in den Figuren 1 bis 3 gesehen - in Richtung zum Stopfen 11 öffnet. Eine derartige Lippe 35 legt sich bei einer Verlagerung des Rings 29 in Figur 2 nach rechts an. Sollte sich der Ring 29 nach links bewegen wollen, würde die Lippe 35 abgespreizt und einer derartigen Bewegung entgegenwirken.

Im Übrigen zeigt sich, dass das Anzeigeelement 21 auch andere Grundformen aufweisen kann, insbesondere dann, wenn es vor Anbringung einer Kolbenstange am Stopfen 11 entfernt wird. Beispielsweise kann ein entlang einer Durchmesserlinie verlaufender Steg vorgesehen werden, dessen Längskanten mit der Innenwand des Spritzenzylinders 1 eine gewisse Reibungskraft aufbauen. Auch können Y-, X- oder sternförmig ausgebildete Körper als Anzeigeelement 21 verwendet und in den Innenraum 9 eingebracht werden.

Besonders bevorzugt wird das anhand von Figur 4 erläuterte Anzeigeelement, weil durch den von dem Ring 29 umschlossenen Freiraum eine Kolbenstange in den Innenraum 9, insbesondere in den Raum 15 eingebracht und in den Stopfen 11 eingeschraubt werden kann. Die Beweglichkeit des Rings 29 wird dadurch nicht beeinträchtigt, insbesondere dann, wenn der Außendurchmesser der Kolbenstange kleiner ist als der Innendurchmesser des Rings 29.

Insgesamt wird deutlich, dass der Spritzenzylinder 1 sich durch die speziell ausgebildete Anzeigeeinrichtung 19 auszeichnet, deren Anzeigeelement 21 ausschließlich in einer Richtung durch den Stopfen 11 verlagerbar ist und in einer einmal erreichten Position verbleibt, um auch bei einer Rückverlagerung des Stopfens 11 dessen Verlagerung eineindeutig anzuzeigen.

Aus den Erläuterungen zu den Figuren 1 bis 3 wurde deutlich, dass der Abstand d zwischen der den Wirkstoffraum 13 zugewandten Kante des Anzeigeelements 21 und der Hilfslinie 23 anhand der Skala 25 ablesbar ist. Dies kann auch mittels einer optischen Leseeinrichtung erfolgen, sei es mittels einer Kamera, eines Mikroskops oder dergleichen.

Die Ablesung der maximalen Verschiebung des Anzeigeelements 21 kann auch mittels einer Blende erfolgen: Es ist möglich, den Spritzenzylinder 1 mit einem in dessen Längsrichtung verlaufenden Balken oder Ring zu versehen, der lichtundurchlässig ist. Die in axialer Richtung des Spritzenzylinders 1 gemessene Länge des Balkens oder Rings entspricht dem maximal zulässigen Abstand d, um den das Anzeigeelement 21 bei einer Verschiebung des Stopfens 11 verlagert werden darf. Die dem Wirkstoffraum 13 zugewandte Kante des Anzeigeelements 21 wird von dem Balken oder dem Ring abgedeckt, wenn bei einer Verlagerung des Anzeigeelements 21 der maximale Abstand d nicht überschritten wird. Sollte dies jedoch der Fall sein, wird zwischen der dem Wirkstoffraum 13 zugewandten Kante des Anzeigeelements 21 und der dem Wirkstoffraum 13 abgewandten Kante des Balkens oder Rings ein Lichtspalt erkennbar, der die unzulässige Verlagerung des Stopfens 11 beziehungsweise des Anzeigeelements 21 eineindeutig anzeigt.

## Patentansprüche

1. Spritzenzylinder mit einem Innenraum (9), mit einem im Innenraum (9) verlagerbaren Stopfen (11), der einen Wirkstoffraum (13) abgrenzt, und mit einer eine in einer Richtung erfolgende Längsbewegung des Stopfens (11) eineindeutig anzeigenden Anzeigeeinrichtung (19), **dadurch gekennzeichnet, dass** die Anzeigeeinrichtung (19) ein Anzeigeelement (21) aufweist, das im Innenraum (9) des Spritzenzylinders (1) gegen eine Reibungskraft beweglich ist, wobei die Reibungskraft so ausgelegt ist, dass sich der Stopfen (11) bei einem Überdruck im Wirkstoffraum (13) mit dem Anzeigeelement (21) in Längsrichtung des Spritzenzylinders (1) verlagern kann, und dass das Anzeigeelement (21) bei Rückverlagerung des Stopfens (11) lagefest im Innenraum (9) verbleibt.

2. Spritzenzylinder nach Anspruch 1, **dadurch gekennzeichnet, dass** das Anzeigeelement (21) als -vorzugsweise einen Schlitz (31) aufweisenden- Ring (29) ausgebildet ist.

3. Spritzenzylinder nach Anspruch 1 oder 2, **dadurch gekennzeichnet, dass** das Anzeigeelement (21) mindestens einen Steg aufweist, dessen Breite im Wesentlichen dem Innendurchmesser des Innenraums (9) entspricht.

4. Spritzenzylinder nach Anspruch 3, **dadurch gekennzeichnet, dass** das Anzeigeelement (21) als Kreuz mit mindestens drei Stegen ausgebildet ist.

5. Spritzenzylinder nach einem der vorhergehenden Ansprüche 1 bis 4, **gekennzeichnet durch** eine die Verlagerung des Anzeigeelements (21) gegenüber dem Stopfen (11) darstellende Messeinrichtung.

6. Spritzenzylinder nach Anspruch 5, **dadurch gekennzeichnet, dass** die Messeinrichtung eine Skala (25) umfasst.

7. Spritzenzylinder nach Anspruch 5, **dadurch gekennzeichnet, dass** die Messeinrichtung eine Blende aufweist.

## Claims

1. A syringe cylinder with an interior space (9), with a stopper (11) that can be displaced in the interior space (9) and delimits an active ingredient space (13), and with an indicator device (19) biuniquely indicating a longitudinal movement of the stopper (11) occurring in one direction, **characterized in that** the indicator device (19) comprises an indicator element (21), which is movable in the interior space (9) of the syringe cylinder (1) against a friction force, the friction force being such that, if overpressure is present in the active ingredient space (13), the stopper (11) can be displaced with the indicator element (21) in the longitudinal direction of the syringe cylinder (1), and that the indicator element (21) remains fixed in the position thereof in the interior space (9) during a return displacement of the stopper (11).

2. The syringe cylinder according to claim 1, **characterized in that** the indicator element (21) is configured as a ring (29), the ring preferably having a slot (31).

3. The syringe cylinder according to claim 1 or 2, **characterized in that** the indicator element (21) has at least one web, the width of which corresponds substantially to the inside diameter of the interior space (9).

4. The syringe cylinder according to claim 3, **characterized in that** the indicator element (21) is configured as a cross having at least three webs.

5. A syringe cylinder according to one of the preceding claims 1 to 4, **characterized by** a measuring device depicting the displacement of the indicator element (21) relative to the stopper (11).

6. The syringe cylinder according to claim 5, **characterized in that** the measuring device comprises a scale (25).

7. The syringe cylinder according to claim 5, **characterized in that** the measuring device comprises an aperture.

## Revendications

1. Cylindre de seringue avec un espace intérieur (9), avec un bouchon (11) pouvant se déplacer dans l'espace intérieur (9) qui délimite un espace de substance active (13), et avec un indicateur (19) indiquant de façon biunivoque un mouvement longitudinal du bouchon (11) dans un sens, **caractérisé en ce que** l'indicateur (19) présente un élément d'affichage (21) qui se déplace dans l'espace intérieur (9) du cylindre de seringue (1) contre une force de frottement, la force de frottement étant conçue de telle sorte que le bouchon (11) peut se déplacer lors d'une surpression dans l'espace de substance active (13) avec l'élément d'affichage (21) dans le sens longitudinal du cylindre de seringue (1), et que l'élément d'affichage (21) reste bien en position dans l'espace intérieur (9) lors du repositionnement du bouchon (11).

2. Cylindre de seringue selon la revendication 1, **caractérisé en ce que** l'élément d'affichage (21) est conçu sous forme d'anneau (29) - de préférence présentant une fente (31).

3. Cylindre de seringue selon la revendication 1 ou 2, **caractérisé en ce que** l'élément d'affichage (21) présente au moins une âme dont la largeur correspond sensiblement au diamètre intérieur de l'espace intérieur (9).

4. Cylindre de seringue selon la revendication 3, **caractérisé en ce que** l'élément d'affichage (21) est conçu sous forme de croix avec au moins trois âmes.

5. Cylindre de seringue selon l'une des revendications précédentes 1 à 4, **caractérisé par** un dispositif de mesure représentant le déplacement de l'élément d'affichage (21) par rapport au bouchon (11).

6. Cylindre de seringue selon la revendication 5, **caractérisé en ce que** le dispositif de mesure comprend une graduation (25).

7. Cylindre de seringue selon la revendication 5, **caractérisé en ce que** le dispositif de mesure présente un diaphragme.
